# EUROPEAN PATENT SPECIFICATION

(11) **EP 0 317 074 B1**
(45) Date of publication and mention of the grant of the patent: **18.12.1996**
(21) Application number: 88309414.6
(22) Date of filing: 07.10.1988
(51) Int. Cl.: C12Q 1/68

(54) **Hybridization method and reagent kit therefor**
Hybridisierungsverfahren und Reagenzsatz dafür
Procédé relatif à l'hybridation et trousse de réactif pour sa mise en oeuvre

(30) Priority: 09.10.1987 FI 874466
(43) Date of publication of application: 24.05.1989
(73) Proprietor: ORION-YHTYMÄ OY, 02101 Espoo (FI)
(72) Inventor: Söderlund, hans Erik, SF-02940 Espoo (FI); Ranki, Tuula Marjut, SF-02660 Espoo (FI); Syvänen, Ann-Christine Elizabeth, SF-00270 Helsinki (FI); Buchert, Petri Uolevi, SF-02230 Espoo (FI); Järvinen, Anne Maritta, SF-00510 Helsinki (FI); Karlsson, Stefan Henrik, SF-02230 Espoo (FI); Korpela, Kai Mikael, SF-00140 Helsinki (FI); Laaksonen, Matti Heikki Sakari, SF-00400 Helsinki (FI); Lönnqvist, Gunnel Solveig Kristina, SF-02760 Espoo (FI); Tenhunen, Jukka Tapani, SF-00500 Helsinki (FI); Weckman, Arja Marjatta, SF-00200 Helsinki (FI)
(74) Representative: Woods, Geoffrey Corlett

(56) References cited:
- EP-A- 0 139 489
- EP-A- 0 200 113
- EP-A- 0 217 403
- EP-A- 0 233 053
- EP-A- 0 237 833
- WO-A-88/04428
- GB-A- 2 202 328

## Description

The invention relates to a method for improving hybridization by means of filtration. The invention also relates to a kit for use in the method and a reagent package.

The sandwich hybridization technique used in the method is described in European Patent EP 79139 and the basic principle of solution hybridization is described for example in British Patent GB 2169403. Solution hybridization is carried out in a solution phase by using at least two mutually nonhomologous probes capable of hybridizing with the target nucleic acid, at least one of the probes, the detector probe, being provided or capable of being provided with a detectable label, and at least one, the capturing probe, being capable of being attached through the formation of an affinity pair to another component, which is fixed to a solid carrier.

In the method according to the invention it is also possible to use solution hybridization in which the detector probe or the capturing probe is replaced by a modified primer. The use of modified primers in hybridization is described in GB-A-2202328. The method uses two modified primers complementary to the opposite polarities of the target nucleic acid. In addition there is needed at least one nucleic acid probe which is capable of hybridizing selectively with the target nucleic acid and is not homologous to the above-mentioned primers. If a modified primer acts as the detector probe, i.e. it is provided or can be provided with a detectable label, the selective probe in question is similar to the capturing probe used in solution hybridization, i.e. it is provided or can be provided with one moiety of an affinity pair or a reaction pair. If a modified primer acts as the capturing probe, the selective probe respectively acts as the detector probe, i.e. it is provided or can be provided with a detectable label.

In the method, the modified primers are allowed to hybridize with the complementary strands, which have been rendered single-stranded, of the target nucleic acid. Thereafter the mixture is incubated in the presence of a polymerase which recognizes the template, i.e. the target nucleic acid molecule; owing to the polymerase the primers may extend into chains comprising up to several thousand bases. The polymerization reaction is repeated when necessary, until the number of target nucleic acid molecule copies required for achieving the desired sensitivity in the given case is obtained, each of the copies being provided with one modified primer. In the event that the target nucleic acid is a single-stranded RNA, in the initial phase a reverse transcriptase is used, with the aid of which a cDNA strand is first prepared. After the polymerization, hybridization is carried out between the copies of the target nucleic acid molecules, provided with modified primers, and the selective probe.

Sandwich hybridization is carried out by using at least two mutually non-homologous probes capable of hybridizing with the target nucleic acid, one of the probes, the capturing probe, being fixed to a solid carrier and the other, the detector probe, being provided or capable of being provided with a detectable label. It is also possible to use solid carrier hybridization in which the modified primer acts as the detector probe (GB-A-2202328). In this case the nucleic acid probe used in the method, capable of hybridizing selectively with the target nucleic acid, is fixed to a micro-particle.

In both the sandwich hybridization method and the solution hybridization method, any excess detector probe is separated from the hybrid formed between the target nucleic acid, the capturing probes and the detector probes, by washing carefully the hybrids bound to the solid carrier (filter, dipstick, microtitre plate, etc.). In the event that micro-particles are used as the solid carrier, the separation is carried out by centrifuging and washing the particles several times, as described in the publication on solution hybridization, Nucleic Acids. Res. 14, 5037-5048, 1986, and in European Patent Specification EP 205532 concerning sandwich hybridization. It is also possible to pack the micro-particles into a chromatography column and elute the excess detector probe from the column (GB 2169403). When modified primers are used, the separation is carried out by the same methods as in sandwich and solution hybridization methods.

EP-A-139489 describes a sandwich hybridisation method for nucleic acid detection in which a biotinylated nucleic acid probe is used in the detection of genes or base sequences of interest in nucleic acids. A preferred embodiment employs enzyme labelled nucleic acid probe for hybridisation with the nucleic acid, a biotinylated nucleic acid probe for specific hybridisation with the gene or base sequence of interest and an avidin coated microparticle for immobilisation thereof. Following separation, label is detected in the bound species for determining the presence of nucleic acid having the desired gene or base sequence. The separation of bound and unbound material may be by centrifiguation, filtration or washing steps. There are no specific examples of the method.

EP-A-192168 describes a dual hybridisation assay method for detecting the presence of a particular polynucleotide sequence in a test sample wherein hybridisation is accomplished in a solution phase. Two soluble polynucleotide probes are employed which hybridise to mutually exclusive portions of the sequence to be detected. The first, or separation, probe comprises a reactive site for a reaction partner, preferably a binding site (e.g. biotin) for a binding substance (e.g. avidin). After hybridisation, the resulting solution is contacted with an immobilised form of the reaction partner whereby dual hybrids comprising the sequence to be detected and the detection and separation probes become immobilised. The immobilised fraction is then separated and then the detection probe determined in one of the separated fractions. The separation of the immobilised fraction from the remaining material may be carried out by centrifugation, filtration, chromatography or decanting. Filtration was not used in the specific examples.

EP-A-200113 describes a method of solid phase nucleic acid hybridisation assay which incorporates a first fragment of nucleic acid bound to a solid carrier comprising water-insoluble particles and a second fragment of nucleic acid bound to a luminescent label. Hybridisation occurs while the solid carrier is suspended and the luminescence is measured after the solid carrier is concentrated by microfiltration.

It is an object of the invention by means of two or more filtrations in the same container to speed up and facilitate the carrying out of hybridization methods and, additionally, to improve their sensitivity, reproducibility, and economy. By the method according to the invention it is also possible to carry out, without cumbersome preliminary treatment steps, nucleic acid assays on samples which contain large amounts of biological impurities difficult to remove.

In the method according to the invention, suitably pretreated particles are used as the solid carrier. Several problems have surfaced in the separation of the particles from the hybridization mixture when conventional procedures have been used, especially when the sample has contained large amounts of insoluble biological impurities. In centrifugation methods, these impurities cannot be separated from the hybrids to be determined; they separate out from the hybridization solution together with the particles. In addition, a portion of the particles are lost in connection with repeated washes, as they adhere to the walls of the centrifuge and the washing containers. The use of chromatography columns, for its part, especially in routine assays, is slow and cumbersome, and also expensive.

The present invention provides a method of detecting a nucleic acid by hybridization comprising:
(a) filtering into a container a solution in which the presence of target nucleic acid or copy of target nucleic acid molecule is to be detected to remove insoluble impurities from the solution,
(b) in said container producing from the filtrate a conjugate of an insoluble particle and hybridized target nucleic acid or hybridized copy of target nucleic acid molecule, the hybridized nucleic acid or copy molecule being attached to the particle via a nucleic acid probe or optionally, in the case of a hybridized copy molecule, via a primer by means of which the copy was made;
(c) in said container separating the conjugate from any excess probe and/or primer by filtration and washing; and
(d) detecting the amount of conjugate present.

An example of a suitable container is a syringe.

According to the invention the method may be carried out by filtering a hybridized or unhybridized nucleic acid sample into a container, in which either the target nucleic acid possibly present in the sample is hybridized when necessary and fixed to the particles in the container or to particles added into the container after the hybridization by mediation of a nucleic acid probe, or copies of the target nucleic acid molecules are filtered into a container, in which the copies of the target nucleic acid molecules are hybridized when necessary and fixed to particles in the container or to particles added into the container after the hybridization by mediation of the modified primers by means of which they were polymerised, and by filtering the particles thereafter and by washing them a suitable number of times in the same container, and by detecting the target nucleic acid by a method suitable in the given case.

The fixing of the target nucleic acid to the particles is carried out, for example, by allowing the target nucleic acid to hybridize with a nucleic acid probe fixed to the particle or with a probe which can be fixed to the particle, or by allowing the nucleic acid probe which has hybridized with the target nucleic acid to become fixed to the particles, or by fixing copies of the target nucleic acid molecules to the particles by mediation of the modified primers by means of which they were polymerized.

By using the method according to the invention, the insoluble biological impurities present in the sample can be conveniently eliminated. The advantages of this method over the centrifugation and chromatography methods are the ease and speed of the washes. Furthermore, the washes are effective since the particles resuspend immediately and completely into the washing solution. Loss of particles, which always occurs in the centrifugation method, is prevented as the filtrations take place in the same container. This improves the sensitivity and reproducibility of the method.

The invention also relates to a kit and a reagent package for use in the method in question. The kit includes a container, advantageously a disposable syringe, in which there are placed the particles pretreated in a suitable manner and at least one filter, which is attached or can be attached to the container. The filter is selected so that the target nucleic acid, the detector and capturing probes and the hybrids will pass through it, but insoluble biological impurities and particles will not. The filter may also be a filter system for example a filter cassette, inside which the particles have been placed in advance. The reagent package includes, in addition to the above-mentioned kit, also the probes and primers needed in the given case, suitably pretreated. It may also include other reagents needed in the method, with their packages, for example reagents used for detecting the detector probe.

The most important hybridization methods to which the method according to the invention can be applied are described below in greater detail. However, the use of the method according to the invention is not limited to the methods described below; it is evident that an expert in the art can apply the principle of the method also to other hybridization methods.

### 1. Solution hybridization improved by means of filtration

Hybridization is carried out in solution in conditions which have been described previously (GB 2169403). After the hybridization, the reaction solution is filtered, at which time the hybrids and the excess detector probe and capturing probe pass through the filter, but any insoluble biological impurities possibly present in the hybridization solution are left outside. The hybrids remaining in the filter can, when necessary, be recovered by rinsing the filter with a hybridization solution not containing nucleic acids. Thereafter the hybrids are allowed to become fixed to particles under conditions which are selected on the basis of the affinity pair used in the method.

Alternatively, the hybridization can be carried out after the filtration. In this case it is most advantageous not to add the particles until after the hybridization, since adding the particles before the hybridization may have a detrimental effect on the kinetics of the hybridization.

After the fixing, the particles are separated from the hybridization solution by filtration, and any excess detector probe is washed away from the particles by filtering the washing liquid through a filter the desired number of times.

The further steps connected with the detection of hybrids depend on the detection method selected in the given case. When a radioactive detector probe is used, the measuring can be done directly from the particles or the filters, or the detector probe can be detached from the particles by using, for example, sodium hydroxide.

Sodium hydroxide elution serves as an additional wash, increasing the sensitivity of the assay, and it is easy to carry out by filtration. If enzymatic detection is used, the dye solution is separated from the particles after the reaction and is measured.

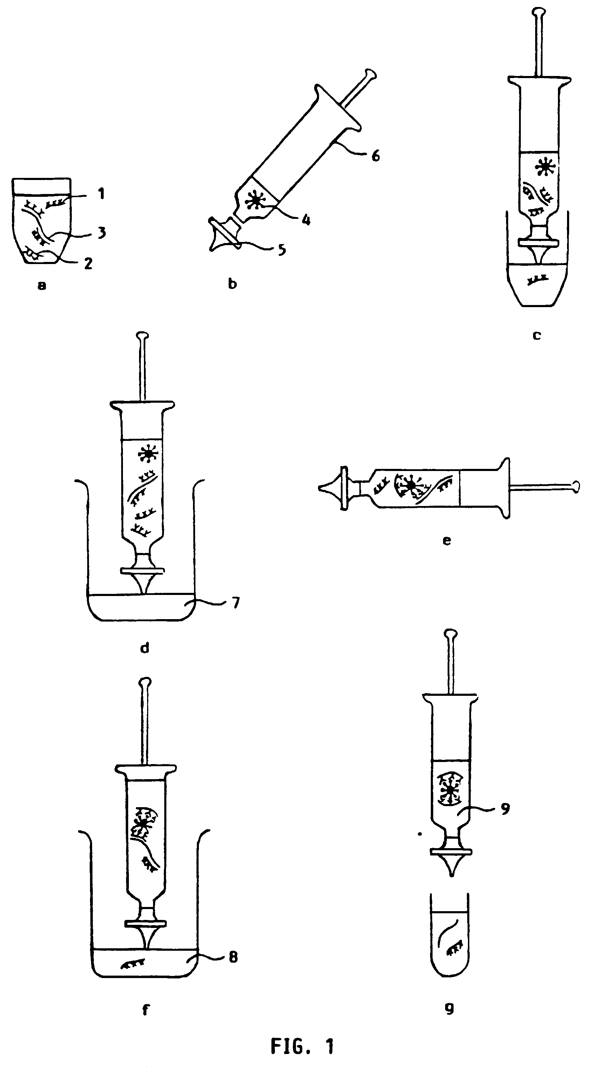

One way of carrying out the method is depicted in figure series a-g (Figure 1), in which the hybridization solution is filtered by means of a syringe.

The steps of the method are:
Step a Hybridization of the detector probes (1), capturing probes (2) and the target nucleic acid (3) is carried out in a separate container.
Step b When necessary, particles (4) are added and a filter (5) is connected to the syringe (6).
Step c The hybridization solution is drawn into the syringe.
Step d When necessary, the filter is rinsed by drawing through it a hybridization solution (7) not containing nucleic acids.
Step e The hybrids are fixed to the particles under conditions advantageous for the formation of an affinity pair.
Step f The hybridization solution is separated from the particles by emptying the syringe, and the particles in the syringe are washed free of unhybridized detector probes by using a washing liquid (8).
Step g The detector probes are eluted from the particles into an eluent (9), and the detector probes are detected from the supernatant. Alternatively, step g can be replaced by assaying the detector probes directly from the particles or the filters.

The reagent package contains at least two mutually nonhomologous probes capable of hybridizing with the target nucleic acid, at least one of the probes, the detector probe, being provided or capable of being provided with a label and at least one, the capturing probe, being provided with a site or a component, or capable of being provided with a component, having affinity for another component. In addition, the package contains at least one filter and a container, preferably a syringe, which has or to which it is possible to connect the said filter, and particles to which the above-mentioned other component has been fixed.

### 2. A solution hybridization method which has been improved by means of filtration and in which a modified primer is used as the detector probe

The target nucleic acid molecules are copied, as described previously (patent application GB-A-2202328), by using two primers which are complementary to the opposite polarities of the target nucleic acid and are provided or capable of being provided with a detectable label. Thereafter hybridization is carried out between the target nucleic acid molecule copies provided with modified primers and the capturing probe which is capable of hybridizing selectively with them and is provided with a site or a component, or capable of being provided with a component, having affinity for another component. The capturing probe must not be homologous to the modified primers used in the method. The hybridization solution is filtered into a container, preferably a syringe, in which the fixing to particles is due to take place. The filter is washed, when necessary, whereafter the hybrids are allowed to fix to the particles under suitable conditions. If the hybridization is not carried out until after the filtration, the particles are preferably added only after the hybridization.

After the fixing, the particles are separated from the hybridization solution and any excess modified primer by filtering and washing the particles the desired number of times. The detection can be carried out either directly from the particles or the filter, or by eluting the copies of the target nucleic acid molecules, provided with modified primers, from the particles and by assaying the label from the supernatant.

The reagent package includes two primers, which are complementary to the opposite polarities of the target nucleic acid and are provided or capable of being provided with a detectable label, and a nucleic acid probe, i.e. a capturing probe, which is capable of hybridizing selectively with the target nucleic acid and which is provided with a site or a component, or capable of being provided with a component, having affinity for another component and is not homologous to the above-mentioned modified primers. In addition, the package includes at least one filter and a container, advantageously a syringe, which has or to which it is possible to connect the said filter, and particles to which the above-mentioned other component has been fixed.

### 3. A solution hybridization method, improved by means of filtration, using a modified primer as the capturing probe

The target nucleic acid molecules are copied by the procedure described previously (patent application GB-A-2202328) by using two primers, complementary to the opposite polarities of the target nucleic acid and provided with a site or component, or capable of being provided with a component, having affinity for another component. Hybridization is carried out between the target nucleic acid molecule copies provided with modified primers and the detector probe capable of hybridizing selectively with them and provided or capable of being provided with a detectable label. The detector probe must not be homologous to the modified primers used in the method. The hybridization solution is filtered into a container, advantageously a syringe, the filter is washed, when necessary, and the target nucleic acid molecule copies provided with modified primers are fixed to the particles. If the hybridization is not carried out until after the filtration, the particles are added into the container preferably only after the hybridization.

After the fixing, the particles are separated from the hybridization solution and any excess detector probes by filtering and washing them a desired number of times, and detection is carried out as described in point 1 above.

The reagent package contains two primers, which are complementary to the opposite polarities of the target nucleic acid and are provided or capable of being provided with a site or component, or capable of being provided with a component, having affinity for another component, and a nucleic acid probe, i.e. a detector probe, which is capable of hybridizing selectively with the target nucleic acid and which is provided or capable of being provided with a detectable label and is not homologous to the modified primer. In addition to these, the package contains at least one filter and a container, advantageously a syringe, which has or to which it is possible to connect the said filter and particles to which the above-mentioned other component has been fixed.

### 4. A sandwich hybridization method improved by means of filtration

A hybridization solution containing mutually pre-hybridized or preferably unhybridized target nucleic acid molecules and detector probes is filtered in order to remove any insoluble biological impurities. The target nucleic acid molecules remaining in the filter can, when necessary, be recovered by rinsing the filter with a hybridization solution which does not contain nucleic acids. Thereafter, hybridization is carried out between the target nucleic acid and the capturing probes fixed to particles, and at the same time hybridization between the target nucleic acid and the detector probes if pre-hybridization has not been carried out. Finally the particles are separated from the hybridization solution by filtration, and detection is carried out either directly from the particles or from the washing solution, as described above regarding solution hybridization.

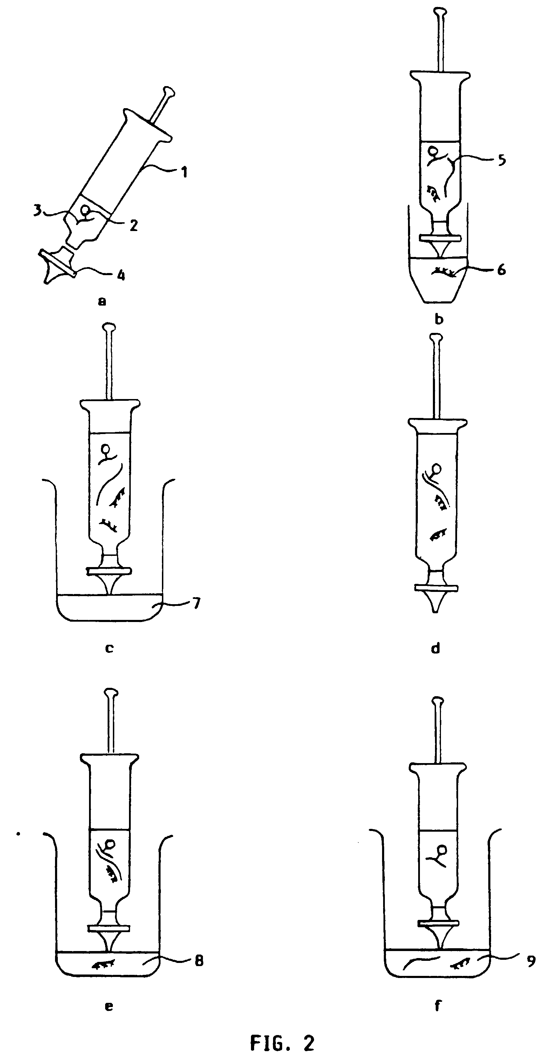

The figure series a-f (Figure 2) depicts a method using a syringe.
Step a When necessary, particles (2) to which the capturing probe (3) has been fixed are introduced into the syringe. The filter (4) is also installed.
Step b The hybridization solution containing the target nucleic acid (5) and the detector probe (6) is drawn into the syringe.
Step c When necessary, the filter is rinsed by drawing through it a hybridization solution (7) which does not contain nucleic acid.
Step d Hybridization is carried out.
Step e Hybridization solution is separated from the particles by emptying the syringe, and the particles in the syringe are washed free of unhybridized probes by using a washing liquid (8).
Step f The detector probes are eluted from the particles into an eluent (9), and the detector probes are detected from the supernatant.

The above-described implementation is merely indicative, and with the aid of this, an expert in the art will be able to devise alternative application methods. Step a can be omitted entirely if the particles and the filter have been fixed in advance. Before step c it is possible to carry out a pre-hybridization in which the target nucleic acid and the detector probe are hybridized with each other. As in solution hybridization, step f can be replaced by assaying the detector probe directly from the particles.

The reagent package to be used in the method contains at least two mutually non-homologous probes capable of hybridizing with the target nucleic acid, at least one of them, the detector probe, being provided or capable of being provided with a detectable lable, and at least one, the capturing probe, being fixed to the particles. In addition, the package contains at least one filter and a container, preferably a syringe, which has, or to which it is possible to connect, the said filter.

### 5. Solid-carrier hybridization, improved by means of filtration, using a modified primer as the detector probe

The target nucleic acid molecules are copied by using two primers complementary to the opposite polarities of the target nucleic acid and provided or capable of being provided with a detectable label. The nucleic acid solution is filtered into a container, advantageously a syringe, and the filter is washed, when necessary, with a hybridization solution which does not contain nucleic acids. Hybridization is carried out between the target nucleic acid molecule copies provided with modified primers and the nucleic acid probe fixed to a particle and capable of hybridizing selectively with the copies. The nucleic acid probe must not be homologous to the modified primers. Next, the particles are separated from the hybridization solution and any excess modified primer by filtering and washing them a desired number of times. The detection is carried out either directly from the particles, or by eluting the target nucleic acid molecule copies provided with modified primers and by assaying the label from the supernatant.

The reagent package contains two primers, which are complementary to the opposite polarities of the target nucleic acid and are provided or capable of being provided with a detectable label, and a nucleic acid probe, i.e. a capturing probe, which is capable of hybridizing selectively with the target nucleic acid and which is fixed to a particle and is not homologous to the above-mentioned modified primers. In addition, the package contains at least one filter and a container, preferably a syringe, which has or to which it is possible to connect the said filter, and particles to which the above-mentioned other component has been fixed.

The practical implementation of the solution hybridization method is described below with the aid of example 1.

### Example 1

Recovery of hybrids in solution hybridization by the Pandex-avidin filtration method.

### Materials

The solid carriers used are polystyrene particles which are avidin-coated (Epicon™ Avidinpolystyrene Assay Particles, Pandex Laboratories, Inc., USA). The diameter of the particles is on the average 0.8 µm, and owing to their small size they remain evenly distributed in the solution. The particles are available as a 5 % (w/v) suspension. To remove any free avidin, the particles are sonicated and washed once before use.

For the filtration of the hybridization solution and the separation of the solid carrier particles from the solution, polyvinyl difluoride filters having a pore size of 0.45 µm (Millex-HV₁₃, Millipore) are used.

The hybrids are fixed to the particles in 1 ml disposable syringes, in which it is convenient to carry out the washes.

### Hybridization

The target DNA was the plasmid pBR-322, linearized using the EcoRI enzyme. The detector probes used were ³²P labelled plasmid pKTH 1300. The capturing probes used were photobiotinylated phages mKTH 1301 and 1302. The preparation of the detector probes and the capturing probes has been described in Journal of Biotechnology, Vol. 5, 267-277, 1987. The hybridization took place in 120 µl of a solution containing in that quantity the following quantities of reagents: target DNA 10⁸, 10⁹ or 10¹⁰ molecules, radioactive probe 4.6 x 10⁵ cpm, i.e. 7 x 10⁸ molecules, and biotinylated mKTH 1301 and 1302 1 x 10¹⁰ molecules each. In addition, the hybridization solution contained sodium chloride 0.6 M, sodium phosphate 20 mM, EDTA 1 mM, and sodium dodecyl sulfate (SDS) 0.1 %. The hybridization period was three hours and the temperature was 65°C.

### Recovery of the hybrids

20 µl of a sonicated, once washed 5 % suspension of Pandex avidin particles was introduced into 1 ml disposable syringes. Thereafter, Millex HV₁₃ filter units were installed in the syringes and 120 µl of the reaction solution was drawn into the syringes. To rinse the hybrids from the filters, 120 µl of DNA-free hybridization solution was additionally drawn into the syringe, whereafter the total volume in the syringes was 260 µl. The fixing of the hybrids to the particles was allowed to take place at 37°C for 15 min in an "end-over-end" mixing apparatus. After the fixing, the particles were washed with a washing solution which contained sodium chloride 15 mM, sodium citrate 1.5 mM, and SDS 0.2 %, and the temperature of which was 50°C. Initially the particles were washed five times by drawing the syringe full (1 ml) of washing solution and by expelling the solution immediately. In addition, they were washed twice by allowing the washing solution to have effect for two minutes each time. After the washes, the detector probe was eluted from the hybrids by drawing into the syringe 125 µl of a solution which contained sodium hydroxide 0.2 M and EDTA 1 mM. The solution was allowed to have effect for 5 min at room temperature, whereafter the treatment was repeated once. 2 ml of Aquasol^{R} scintillation liquid was added to the sodium hydroxide supernantants, and their radioactivity was measured using a beta counter.

### Results

The results can be summarized in the following table:

| Target DNA¹⁾ | specific cpm²⁾ | signal/background |
|---|---|---|
| 10⁸ | 4012 | 8.0 |
| 10⁹ | 27551 | 49.0 |
| 10¹⁰ | 46515 | 82.0 |

| | | |
|---|---|---|
| 1) Number of molecules | | |
| 2) cpm is a mean of three parallel determinations; the signal (573 cpm) given by the 0 sample has been subtracted from the specific cpm. | | |

### Example 2

### Recovery of hybrids in sandwich hybridization on microparticles by filtration

### Reagents

The sandwich hybridization reagents were derived from the 4.2 kb cryptic plasmid (pJD1) of Neisseria gonorrhoeae (Korch, C., Hagblom, P. & Normark, S. (1983). Sequence-specific DNA modification in Neisseria gonorrhoeae. Journal of Bacteriology 155, 1324-32.). The recombinant plasmid Flol/pUMl, containing the N. gonorrhoeae cryptic plasmid in pBR322 was used as the target DNA. The recombinant plasmid pKTH1368, containing a 1.3 kb HinfI-TaqI fragment of the N. gonorrhoeae cryptic plasmid in the plasmid pAT153, was used as the capturing probe. The recombinant phages mKTH1369 and mKTH1370, (both polarities) containing a 1.7 kb HinfI-HinfI fragment in the M13 mp10 phage, were used as the detector probe. The target-DNA (Flol/pUMl) was linearized with EcoRl.

### Labeling

The phages mKTH1369 and mKTH1370, used as detector probes, were labeled with [α-³²P]dCTP (Amersham, U.K.) by primer extension with the Klenow fragment of DNA polymerase I to a specific activity of about 10⁸ cpm/µg. The plasmid pKTH1368, used as a tracer in quantifying the amount of bound plasmid DNA to the microparticles, was labeled by nick translation (12) with [α-³²P]dCTP to a specific activity of about 10⁸ cpm/µg.

Coupling of capturing probes to microparticles.

The capturing probe was attached to uniform polystyrene microparticles (0-4.5 µm) containing a secondary hydroxy groups on their surface. The hydroxygroups on the surface of the particles were activated by addition of 0.29 g (1.5 mmol) p-toluenesulfonyl chloride and 60 µl (0.75 mmol) pyridine to 30 mg particles in 1 ml acetone with end-over-end incubation at room temperature for 20 h. The particles were transferred to aqueous solution by washing with increasing H₂O concentrations in acetone. Finally the particles were washed once with 0.2 M borate buffer pH 9.5 and then suspended in the same buffer to a concentration of 30 mg/ml. The plasmid pKTH1368 was denatured and fragmented by boiling in 0.2 M NaOH for 5 min. 10 µg of pKTH1368 was incubated with one mg of activated beads in 75 µl 0.2 M borate buffer pH 9.5 at room temperature for 18 h with end-over-end rotation. The particles were separated from the solution by centrifugation. The amount of coupled DNA was quantified using radioactively labeled pKTH1368 as tracer. The unreacted p-toluenesulfonyl groups were blocked with 1 M Tris-HCl buffer, pH 9.0 at room temeprature for 4 hours. The beads were suspended in 0.05 M Tris-HCl, pH 7.5, 0.1 M NaCl and 0.01 % sodium dodecyl sulfate (SDS) to a concentration of 30 mg/ml and stored at +4°C.

### Hybridization and recovery of hybrids

The hybridization reaction was carried out in 1 ml disposable syringes containing Millex HV13 filter units (filters as in Example 1), introducing into syringes one mg of particles per assay, which corresponds to 3.7 x 10¹⁰ molecules of capturing probe (61 fmol). As detector probes 2 x 10⁸ molecules 300 000 cpm (0.3 fmol) of ³²P-labeled mKTH1369 and mKTH1370 were used per reaction. 70 µl of reaction solution containing 0.6 M NaCl, 60 mM sodium citrate, pH 7.6 (4 x SSC), 20mM sodium phosphate and 0,25 % SDS, detector probes and in the table below indicated amounts of target DNA was drawn into the syringes. To rinse the target DNA from the filters 30 µl of DNA-free hybridization solution was additionally drawn into the syringe, whereafter the total volume in the syringes was 100 µl. The hybridization period was 5 hours and the temperature was 65°C.. After the hybridization the particles were washed five times at 55 °C with 0.1 x 55C, 0.2 % SDS by sucking the solution through the filter. After the washes, the elution of the hybrids and the detection was performed as previously described (see example 1).

### Results

The results can be summarized in the following table:

| Target DNA* | Specific cpm** | signal/backround |
|---|---|---|
| 4x10⁷ | 762 | 8.5 |
| 10⁶ | 963 | 10.7 |
| 4x10⁸ | 6877 | 76.4 |
| 10⁹ | 12925 | 143.6 |

| | | |
|---|---|---|
| *) number of molecules | | |
| **) CPM is a mean of four parallel determinations ; the signal (90 cpm) given by the 0 sample has been subtracted from the specific cpm. | | |

### Example 3

### Recovery of hybrids in solution hybridization by the Pandex-avidin filtration method using a modified primer as the detector probe

### Materials and reagents

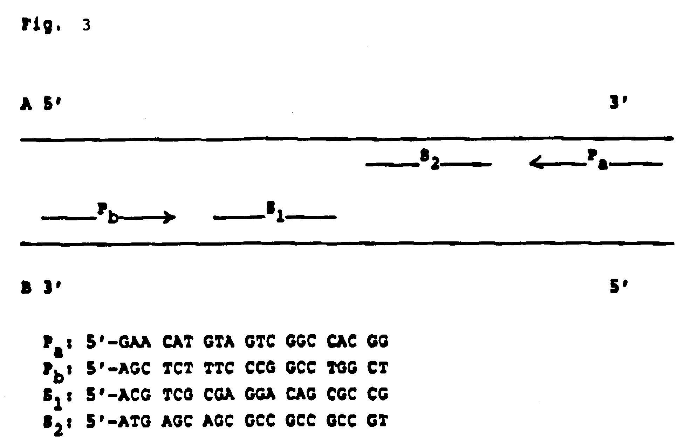

The solid carriers, filters and container used were as in example 1. In this model experiment the target was a recombinant plasmid (pBR322/CMV HindIII L) which harbours a 12.3 kb fragment of the cytomegalovirus (CMV, AD 169, ATCC VR-538) genome. The two detector primers (Pₐ, P_{b}, Fig. 3) used were 20 nucleotides long and synthesized with standard methods on an automated synthesizer. They correspond to two regions on the CMV-specific insert which were 111 nucleotides apart. Two selective capturing probes (S₁, S₂, Fig. 3) recognized regions on each of the two strands between the two detector primers. The detector primers Pₐ P_{b} were labelled with ³²P at their 5′ ends to a specific activity of 4 x 10⁹ CPM/µg using the well known reaction with polynucleotide kinase and gamma-³²P-ATP (Maniatis et al., Molecular Cloning, A Laboratory Manual, Cold Spring Harbor Laboratory, 1982).

Biotinylated nucleotides were added to the 3′ ends of the capturing probes using bio 11-dUTP (BRL) and terminal transferase (Promega Biotech.) as described by Riley et al., DNA. 5 (4), pp. 333-337, 1986. The target plasmid was linearized using the enzyme EcoRI. DNA polymerase, Klenow fragment was purchased from Boehringer-Mannheim.

### Copying the target DNA

Four different reactions were assembled containing 0, 10⁴, 10⁶ and 10⁸ molecules (corresponding to 2 x 10⁻²⁰, 2 x 10⁻¹⁸ and 2 x 10⁻¹⁶ moles) respectively of the target DNA. In addition all four reactions contained in a total volume of 50 µl: 2 pmol each of the two primers, 0.5 mM of each of the four deoxynucleoside triphosphates (i.e. dATP, dCTP, dGTP and dTTP), 10 mm Tris-CL pH 7,5 10 mM MgCl₂, 50 mM NaCl and 10 mM dithiothreitol. The mixture was heated to 100°C for 2 min, then incubated for 5 min at 37°C, whereafter 1 µl (equalling 1 unit) of DNA-polymerase was added. Then the mixture was again incubated for 10 min at 37°C. The boiling followed by annealing of the detector primers and an incubation with added DNA polymerase at 37°C constitutes a DNA synthesizing cycle. The cycle was repeated 16 times.

### Hybridization

After the last cycle the sample was again heated to 100°C whereafter 10 pmole of the selective capturing probe was added together with NaCl, (0,9 M) EDTA (2 mM) sodium phosphate pH 7,5 (20 mM) and sodium dodecyl sulphate (0.1 %).

The volume increased to 100 µl and the concentrations given are as final concentrations. The hybridization period was 1 hour and the temperature was 50°C.

### Recovery of the hybrids

Pandex-avidin particles were introduced into 1 ml disposable syringes and millex HV₁₃ filter units were installed in the syringes as in Example 1. 100 µl of the reaction solution was drawn into the syringes. To rinse the hybrids from the filter, 120 µl of DNA-free hybridization solution was additionally drawn into the syringe, whereafter the total volume in the syringes was 240 µl. The fixing of the hybrids to the particles and washing the particles were performed as in Example 1. After the washes the copies of the target nucleic acid molecules provided with modified primers were detected as in Example 1. Results The results can be summarised in the following table:

| target DNA (moles) | ³²P-activity in collected hybrids ^{a)} (CPM above background) ^{b)} |
|---|---|
| | 16 No. of cycles |
| 0 | 0 |
| 2 x 10⁻²⁰ | 1050 |
| 2 x 10⁻¹⁸ | 15000 |
| 2 x 10⁻¹⁶ | 51300 |

| | |
|---|---|
| a) Mean of two determinations | |
| b) ND - not detectable (less radioactivity than 2 times mean background activity) | |

### Example 4

Recovery of hybrids in solution hybridization by the Pandex-avidin filtration method using a modified primer as the capturing probe.

The target DNA and materials and reagents used were the same as in Example 3 with the following exceptions: The capturinq primers (Pₐ, P_{b}, Fig. 3) were not labelled with ³²P but their 5' ends were instead modified to contain a biotin residue. This chemical modification was done using know methods described by Chollet and Kawashima, Nucleic Acids Research, 13, pp. 1529-1541, 1985. The two selective probes (S₁ and S₂, Fig. 3) were in this case labelled in their 5' ends to act as detector probes. Their specific activities were approximately 2 x 10⁹ and 2,5 x 10⁹ cpm/µg respectively.

The copying of the target DNA and the hybridization were performed as described in Example 3. The biotinylated capturing primers were, however, added in 10 fold amounts, i.e. 10 pmol each per reaction. 16 cycles were performed as described in Example 3 whereafter the sample were heated to 100°C and 0,5 pmol each of the ³²P-labelled probes S₁ and S₂ were added. The hybridization was carried out in the same conditions as described in Example 3.

The hybrids were then collected on Pandex-avidin particles, washed and counted for ³²P-activity, as in Example 3. The results are shown in the following table.

| target DNA (moles) | ³²P-activity in collected hybrids ^{a)} (CPM above background) ^{b)} |
|---|---|
| | 16 No. of cycles |
| 0 | 0 |
| 2 x 10⁻²⁰ | 1420 |
| 2 x 10⁻¹⁸ | 18500 |
| 2 x 10⁻¹⁶ | 68300 |

| | |
|---|---|
| a) Mean of two determinations | |
| b) ND - Not detectable | |

## Claims

1. A method of detecting a nucleic acid by hybridization comprising:
(a) filtering into a container a solution in which the presence of target nucleic acid or copy of target nucleic acid molecule is to be detected to remove insoluble impurities from the solution,
(b) in said container producing from the filtrate a conjugate of an insoluble particle and hybridized target nucleic acid or hybridized copy of target nucleic acid molecule, the hybridized nucleic acid or copy molecule being attached to the particle via a nucleic acid probe or optionally, in the case of a hybridized copy molecule, via a primer by means of which the copy was made;
(c) in said container separating the conjugate from any excess probe and/or primer by filtration and washing; and
(d) detecting the amount of conjugate present.

2. A method according to claim 1 in which the container is a syringe.

3. A method according to claim 2 in which filtration is through a filter attached to the nozzle of the syringe.

4. A method according to claim 1, 2 or 3 which comprises:
(a) hybridizing target nucleic acid if present in the solution with a capturing probe and a detector probe and filtering the hybridized solution optionally recovering hybrid remaining in the filter by rinsing the filter;
(b) fixing the hybrid in the filtrate to an insoluble particle to form hybrid-particle conjugate;
(c) separating the hybrid-particle conjugate from the solution by filtration and washing the conjugate to remove unhybridized detector probe; and
(d) detecting the amount of conjugate present.

5. A method according to claim 1, 2 or 3 which comprises:
(a) filtering the solution in which the presence of target nucleic acid is to be detected, optionally recovering target nucleic acid remaining in the filter by rinsing the filter and then hybridizing target nucleic acid if present in the filtrate with a capturing probe and a detector probe;
(b) fixing the hybrid in the filtrate to an insoluble particle to form hybrid-particle conjugate;
(c) separating the hybrid-particle conjugate from the solution by filtration and washing the conjugate to remove unhybridized detector probe; and
(d) detecting the amount of conjugate present.

6. A method according to claim 1, 2 or 3 which comprises:
(a1) copying any target nucleic acid present using two primers which are complementary to the opposite polarities of the target nucleic acid and which are provided with or are capable of being provided with a detectable label;
(a2) hybridizing the target nucleic acid copies with a capturing probe which is not homologous with either of the primers;
(a3) filtering the hybridized solution to remove insoluble impurities, optionally recovering hybrid remaining in the filter by rinsing the filter;
(b) fixing the hybrid in the filtrate to an insoluble particle via the capturing probe to form hybrid-particle conjugate;
(c) separating the hybrid-particle conjugate from the solution by filtration and washing the conjugate to remove any unhybridized primer; and
(d) detecting the amount of conjugate present.

7. A method according to claim 1, 2 or 3 which comprises:
(a1) copying any target nucleic acid present using two primers which are complementary to the opposite polarities of the target nucleic acid and which are provided with or are capable of being provided with a detectable label;
(a2) filtering the solution of target nucleic acid copies to remove insoluble impurities, optionally recovering target nucleic acid copies remaining in the filter by rinsing the filter;
(a3) hybridizing the target nucleic acid copies in the filtrate with a capturing probe which is not homologous with either of the primers
(b) fixing the hybrid to an insoluble particle via the capturing probe to form hybrid-particle conjugate;
(c) separating the hybrid-particle conjugate from the solution by filtration and washing the conjugate to remove any unhybridized primer; and
(d) detecting the amount of conjugate present.

8. A method according to claim 1, 2 or 3 which comprises:
(a1) copying any target nucleic acid present using two primers which are complementary to the opposite polarities of the target nucleic acid;
(a2) hybridizing the target nucleic acid copies with a detector probe which is provided with or is capable of being provided with a detectable label and which is not homologous with either of the primers;
(a3) filtering the hybridized solution to remove insoluble impurities, optionally recovering hybrid remaining in the filter by rinsing the filter;
(b) fixing the hybrid to an insoluble particle via a primer to form hybrid-particle conjugate;
(c) separating the hybrid-particle conjugate from the solution by filtration and washing the conjugate to remove any unhybridized detector probe; and
(d) detecting the amount of conjugate present.

9. A method according to claim 1, 2 or 3 which comprises:
(a1) copying any target nucleic acid present using two primers which are complementary to the opposite polarities of the target nucleic acid;
(a2) filtering the solution of target nucleic acid copies to remove insoluble impurities, optionally recovering target nucleic acid copies remaining in the filter by rinsing the filter;
(a3) hybridizing the target nucleic acid copies in the filtrate with a detector probe which is provided with or is capable of being provided with a detectable label and which is not homologous with either of the primers;
(b) fixing the hybrid to an insoluble particle via a primer to form a hybrid-particle conjugate;
(c) separating the hybrid-particle conjugate from the solution by filtration and washing the conjugate to remove any unhybridized detector probe; and
(d) detecting the amount of conjugate present. 3

10. A method according to any one of claims 1 to 3 comprising:
(a) filtering a solution comprising detector probe and in which the presence of target nucleic acid is to be detected, optionally recovering target nucleic acid remaining in the filter by rinsing the filter;
(b) hybridizing target nucleic acid if present in the filtrate with a detector probe and a capturing probe, the capturing probe being fixed to an insoluble particle to form a hybrid-particle conjugate;
(c) separating the hybrid-particle conjugate from the solution by filtration and washing the conjugate to remove unhybridized detector probe; and
(d) detecting the amount of conjugate present.

11. A method according to any one of claims 1 to 3 comprising:
(a) pre-hybridizing target nucleic acid if present in the solution with a detector probe and filtering the hybridized solution, optionally recovering pre-hybrid remaining in the filter by rinsing the filter;
(b) hybridizing the pre-hybrid with a capturing probe, the capturing probe being fixed to an insoluble particle to form a hybrid-particle conjugate;
(c) separating the hybrid-particle conjugate from the solution by filtration and washing the conjugate to remove unhybridized detector probe; and
(d) detecting the amount of conjugate present.

12. A method according to any one of claims 1 to 3 comprising:
(a1) copying any target nucleic acid present using two primers which are complementary to the opposite polarities of the target nucleic acid and which are provided with or are capable of being provided with a detectable label;
(a2) filtering the solution of target nucleic acid copies to remove insoluble impurities, optionally recovering target nucleic acid copies remaining in the filter by rinsing the filter;
(b) hybridizing target nucleic acid copies if present in the filtrate with a nucleic acid probe which is not homologous with either primer and which is fixed to an insoluble particle, to form a hybrid-particle conjugate;
(c) separating the hybrid particle conjugate from the solution by filtration and washing the conjugate to remove any unhybridized primer; and
(d) detecting the amount of conjugate present.

13. A method according to any one of claims 4, 5, 8, 9, 10 or 11 in which in step (d) detector probe is eluted from the hybrid-particle conjugate and the amount of probe is assayed.

14. A method according to any one of claims 6, 7 or 12 in which in step (d) target nucleic acid copies provided with a primer provided with a label are eluted from the hybrid-particle conjugate and the amount of label in the supernatant is assayed.

15. Use of a container which contains particles capable of forming a hybrid-particle complex with a hybrid of the target nucleic acid, and at least one filter which is connected or is capable of being connected to the container, as a kit in an assay method as defined in any one of claims 1 to 14

16. Use of at least two mutually non-homologous probes capable of hybridizing with the target nucleic acid, at least one of the probes, the detector probes, being provided or capable of being provided with a detectable label, and at least one, the capturing probe, being provided with a site or a first component, or capable of being provided with a first component, having affinity for a second component, at least one filter, a container to which the filter is connected or to which it is possible to connect the filter, and particles to which the said second component has been fixed, as a reagent package for detecting nucleic acid in a method as defined in any one of claims 1 to 14.

17. Use of two primers, which are complementary to the opposite polarities of the target nucleic acid and are provided or capable of beng provided with a label; a nucleic acid probe, i.e. a capturing probe, which is capable of hybridizing selectively with the target nucleic acid, and which is provided with a site or a first component, or capable of being provided with a first component, having affinity for a second component, and is not homologous to either modified primer; at least one filter, a container to which the filter is connected or to which it is possible to connect the said filter, and particles to which the said second component has been fixed, as a reagent package for detecting nucleic acid in a method as defined in any one of claims 1 to 14.

18. Use of at least two primers, which are complementary to the opposite polarities of the target nucleic acid and are provided or capable of being provided with a site or a first component, having affinity for a second component; a nucleic acid probe, i.e. a detector probe, which is capable of hybridizing selectively with the target nucleic acid and which is provided or can be provided with a detectable label and is not homologous to either primer; at least one filter, a container to which the filter is connected or to which it is possible to connect the said filter; and particles to which the said second component has been fixed, as a reagent package for detecting nucleic acid in a method as defined in any one of claims 1 to 14.

19. Use of at least two mutually non-homologous probes capable of hybridizing with the target nucleic acid, at least one of then, the detector probe, being provided or capable of being provided with a detectable label and the other, the capturing probe, being fixed to particles, and at least one filter and a container to which the filter is connected or to which it is possible to connect the said filter, as a reagent package for detecting nucleic acid in a method as defined in any one of claims 1 to 14.

20. Use of two primers, which are complementary to the opposite polarities or the target nucleic acids and which are provided or capable of being provided with a detectable label; a nucleic acid probe, i.e. a capturing probe, which is capable of hybridizing selectively with the target nucleic acid and which is fixed to particles and is not homologous to either primer; at least one filter and a container to which the filter is connected or to which it is possible to connect the said filter; and particles to which the capturing probe has been fixed, as a reagent package for detecting nucleic acid in a method as defined in any one of claims 1 to 14.

21. A kit suitable for use in assaying nucleic acid in a method according to any one of claims 1 to 14 comprising a syringe which contains particles capable of forming a hybrid-particle complex with a hybrid of the target nucleic acid, and at least one filter which is connected or is capable of being connected to the container.

## Patentansprüche

1. Verfahren zur Bestimmung einer Nucleinsäure durch Hybridisierung umfassend:
(a) Filtern einer Lösung, in der die Gegenwart einer Ziel-Nucleinsäure oder einer Kopie des Ziel-Nucleinsäuremoleküls bestimmt werden soll, in einen Behälter, um unlösliche Verunreinigungen aus der Lösung zu entfernen;
(b) Herstellen eines Konjugats eines unlöslichen Teilchens und einer hybridisierten Ziel-Nucleinsäure oder einer hybridisierten Kopie eines Ziel-Nucleinsäuremoleküls aus dem Filtrat im Behälter, wobei die hybridisierte Nucleinsäure oder die Kopie des Moleküls an das Teilchen über eine Nucleinsäuresonde oder gegebenenfalls, im Fall einer hybridisierten Kopie des Moleküls, über einen Primer, mit dessen Hilfe die Kopie hergestellt wurde, gebunden ist;
(c) Trennen des Konjugats in dem Behälter von überschüssiger Sonde und/oder Primer durch Filtration und Waschen; und
(d) Bestimmen der Menge des vorhandenen Konjugats.

2. Verfahren nach Anspruch 1, dadurch gekennzeichnet, daß der Behälter eine Injektionsspritze ist.

3. Verfahren nach Anspruch 2, dadurch gekennzeichnet, daß die Filtration durch ein an der Düse der Injektionsspritze angebrachtes Filter erfolgt.

4. Verfahren nach Anspruch 1, 2 oder 3, umfassend:
(a) Hybridisieren einer in der Lösung gegebenenfalls vorhandenen Ziel-Nucleinsäure mit einer Einfangsonde und einer Detektorsonde, und Filtrieren der hybridisierten Lösung, und gegebenenfalls Gewinnen des im Filter verbleibenden Hybrids durch Spülen des Filters;
(b) Fixieren des Hybrids im Filtrat auf ein unlösliches Teilchen, um ein Hybrid/Teilchen-Konjugat auszubilden;
(c) Abtrennen des Hybrid/Teilchen-Konjugats aus der Lösung mittels Filtration und Waschen des Konjugats, um unhybridisierte Detektorsonde zu entfernen; und
(d) Bestimmen der Menge des vorhandenen Konjugats.

5. Verfahren nach Anspruch 1, 2 oder 3, umfassend:
(a) Filtrieren der Lösung, in der die Gegenwart der Ziel-Nucleinsäure bestimmt werden soll, gegebenenfalls Gewinnen der im Filter verbleibenden Ziel-Nucleinsäure durch Spülen des Filters, und dann Hybridisieren der gegebenenfalls im Filtrat vorhandenen Ziel-Nucleinsäure mit einer Einfangsonde und einer Detektorsonde;
(b) Fixieren des Hybrids im Filtrat auf einem unlöslichen Teilchen, um ein Hybrid/Teilchen-Konjugat auszubilden;
(c) Abtrennen des Hybrid/Teilchen-Konjugats aus der Lösung mittels Filtration und Waschen des Konjugats, um nicht hybridisierte Detektorsonde zu entfernen; und
(d) Bestimmen der Menge des vorhandenen Konjugats.

6. Verfahren nach Anspruch 1, 2 oder 3, umfassend:
(a1) Kopieren einer vorhandenen Ziel-Nucleinsäure unter Verwendung von zwei Primern, die komplementär zu den gegenüberliegenden Polaritäten der Ziel-Nucleinsäure sind, und die mit einem bestimmbaren Marker versehen oder mit einem solchen versehen werden können;
(a2) Hybridisieren der Ziel-Nucleinsäurekopien mit einer Einfangsonde, die mit irgendeinem der Primer nicht homolog ist;
(a3) Filtrieren der hybridisierten Lösung, um unlösliche Verunreinigungen zu entfernen, und gegebenenfalls Gewinnen des im Filter verbleibenden Hybrids durch Spülen des Filters;
(b) Fixieren des Hybrids im Filtrat auf einem unlöslichen Teilchen mittels der Einfangsonde, um ein Hybrid/Teilchen-Konjugat auszubilden;
(c) Abtrennen des Hybrid/Teilchen-Konjugats aus der Lösung durch Filtration und Waschen des Konjugats, um irgendwelchen nicht-hybridisierten Primer zu entfernen; und
(d) Bestimmen der Menge des vorhandenen Konjugats.

7. Verfahren nach Anspruch 1, 2 oder 3, umfassend:
(a1) Kopieren irgendeiner vorhandenen Ziel-Nucleinsäure unter Verwendung von zwei Primern, die komplementär zu den gegenüberliegenden Polaritäten der Ziel-Nucleinsäure sind, und die mit einem bestimmbaren Marker versehen sind oder damit versehen werden können;
(a2) Filtrieren der Lösung der Ziel-Nucleinsäurekopien, um unlösliche Verunreinigungen zu entfernen, und gegebenenfalls Gewinnen der auf dem Filter verbleibenden Ziel-Nucleinsäurekopien durch Spülen des Filters;
(a3) Hybridisieren der Ziel-Nucleinsäurekopien im Filtrat mit einer Einfangsonde, die mit irgendeinem der Primer nicht homolog ist;
(b) Fixieren des Hybrids auf einem unlöslichen Teilchen mittels der Einfangsonde, um ein Hybrid/Teilchen-Konjugat auszubilden;
(c) Abtrennen des Hybrid/Teilchen-Konjugats aus der Lösung durch Filtration und Waschen des Konjugats, um irgendwelchen nicht-hybridisierten Primer zu entfernen; und
(d) Bestimmen der Menge des vorhandenen Konjugats.

8. Verfahren nach Anspruch 1, 2 oder 3, umfassend:
(a1) Kopieren irgendeiner vorhandenen Ziel-Nucleinsäure unter Verwendung von zwei Primern, die zu den gegenüberliegenden Polaritäten der Ziel-Nucleinsäure komplementär sind;
(a2) Hybridisieren der Ziel-Nucleinsäurekopien mit einer Detektorsonde, die mit einem bestimmbaren Marker versehen ist oder mit einem solchen versehen werden kann, und die mit irgendeinem der Primer nicht homolog ist;
(a3) Filtrieren der hybridisierten Lösung, um unlösliche Verunreinigungen zu entfernen, und gegebenenfalls Gewinnen des in Filter verbleibenden Hybrids durch Spülen des Filters;
(b) Fixieren des Hybrids an einem unlöslichen Teilchen mittels eines Primers, um ein Hybrid/Teilchen-Konjugat auszubilden;
(c) Abtrennen des Hybrid/Teilchen-Konjugats aus der Lösung durch Filtration und Waschen des Konjugats, um irgendeine nicht-hybridisierte Detektorsonde zu entfernen; und
(d) Bestimmung der Menge des vorhandenen Konjugats.

9. Verfahren nach Anspruch 1, 2 oder 3, umfassend:
(a1) Kopieren irgendeiner vorhandenen Ziel-Nucleinsäure unter Verwendung von zwei Primern, die komplementär zu den gegenüberliegenden Polaritäten der Ziel-Nucleinsäure sind;
(a2) Filtrieren der Lösung der Ziel-Nucleinsäurekopien, um unlösliche Verunreinigungen zu entfernen, und gegebenenfalls Gewinnen der im Filter verbleibenden Ziel-Nucleinsäurekopien durch Spülen des Filters;
(a3) Hybridisieren der Ziel-Nucleinsäurekopien im Filtrat mit einer Detektorsonde, die mit einem bestimmbaren Marker versehen ist oder damit versehen werden kann, und die mit irgendeinem der Primer nicht homolog ist;
(b) Fixieren des Hybrids auf einem unlöslichen Teilchen mittels eines Primers, um ein Hybrid/Teilchen-Konjugat auszubilden;
(c) Abtrennen des Hybrid/Teilchen-Konjugats aus der Lösung durch Filtration und Waschen des Konjugats, um irgendeine nicht-hybridisierte Detektorsonde zu entfernen; und
(d) Bestimmen der Menge des vorhandenen Konjugats.

10. Verfahren nach einem der Ansprüche 1 bis 3, umfassend:
(a) Filtrieren einer Lösung, die eine Detektorsonde umfaßt, und in der die Gegenwart einer Ziel-Nucleinsäure bestimmt werden soll, und gegebenfalls Gewinnen der auf dem Filter verbleibenden Ziel-Nucleinsäure durch Spülen des Filters;
(b) Hybridisieren der gegebenenfalls im Filtrat vorhandenen Ziel-Nucleinsäure mit einer Detektorsonde und einer Einfangsonde, wobei die Einfangsonde an einem unlöslichen Teilchen fixiert ist, um ein Hybrid/Teilchen-Konjugat auszubilden;
(c) Abtrennen des Hybrid/Teilchen-Konjugats aus der Lösung durch Filtration und Waschen des Konjugats, um unhybridisierte Detektorsonde zu entfernen; und
(d) Bestimmen der Menge des vorhandenen Konjugats.

11. Verfahren nach einem der Ansprüche 1 bis 3, umfassend:
(a) Pre-Hybridisieren einer gegebenfalls in der Lösung vorhandenen Ziel-Nucleinsäure mit einer Detektorsonde und Filtrieren der hybridisierten Lösung, und gegebenenfalls Gewinnen des im Filter verbleibenden Pre-Hybrids durch Spülen des Filters;
(b) Hybridisieren des Pre-Hybrids mit einer Einfangsonde, wobei die Einfangsonde auf einem unlöslichen Teilchen fixiert ist, um ein Hybrid/Teilchen-Konjugat auszubilden;
(c) Abtrennen des Hybrid/Teilchen-Konjugats aus der Lösung durch Filtration und Waschen des Konjugats, um nicht hybridisierte Detektorsonde zu entfernen; und
(d) Bestimmen der Menge des vorhandenen Konjugats.

12. Verfahren nach einem der Ansprüche 1 bis 3, umfassend:
(a1) Kopieren irgendeiner vorhandenen Ziel-Nucleinsäure unter Verwendung von zwei Primern, die komplementär zu den gegenüberliegenden Polaritäten der Ziel-Nucleinsäure sind und die mit einem bestimmbaren Marker versehen sind oder damit versehen werden können;
(a2) Filtrieren der Lösung der Ziel-Nucleinsäurekopien, um unlösliche Verunreinigungen zu entfernen, und gegebenfalls Gewinnen der im Filter verbleibenden Ziel-Nucleinsäurekopien durch Spülen des Filters;
(b) Hybridisieren der gegebenfalls im Filtrat vorhandenen Ziel-Nucleinsäurekopien mit einer Nucleinsäuresonde, die mit irgendeinem Primer nicht homolog ist, und die an ein unlöslichen Teilchen fixiert ist, um ein Hybrid/Teilchen-Konjugat auszubilden;
(c) Abtrennen des Hybrid/Teilchen-Konjugats aus der Lösung durch Filtration und Waschen des Konjugats, um irgendeinen nicht-hybridisierten Primer zu entfernen; und
(d) Bestimmen der Menge des vorhandenen Konjugats.

13. Verfahren nach einem der Ansprüche 4, 5, 8, 9, 10 oder 11, dadurch gekennzeichnet, daß in Stufe (d) die Detektorsonde vom Hybrid/Teilchen-Konjugat eluiert wird und die Menge der Sonde bestimmt wird.

14. Verfahren nach einem der Ansprüche 6, 7 oder 12, dadurch gekennzeichnet, daß in Stufe (d) die Ziel-Nucleinsäurekopien, die mit einem mit einem Marker versehenen Primer versehen sind, vom Hybrid/Teilchen-Konjugat eluiert werden und die Menge des Markers im Überstand bestimmt wird.

15. Verwendung eines Behälters, der Teilchen, die dazu fähig sind, einen Hybrid/Teilchen-Komplex mit einem Hybrid der Ziel-Nucleinsäure auszubilden, und mindestens ein Filter, das mit dem Behälter verbunden ist oder mit ihm verbunden werden kann, enthält, als Kit (Reagenzsatz) in einem in einem der Ansprüche 1 bis 14 definierten Bestimmungsverfahren.

16. Verwendung von mindestens zwei gegenseitig nicht-homologer Sonden, die dazu fähig sind, mit der Ziel-Nucleinsäure zu hybridisieren, wobei mindestens eine der Sonden, die Detektorsonden, mit einem bestimmbaren Marker versehen ist oder damit versehen werden kann, und mindestens eine, die Einfangsonde, mit einer Stelle oder einer ersten Komponente versehen ist, oder mit einer ersten Komponente versehen werden kann, die Affinität für eine zweite Komponente besitzt, und von mindestens einem Filter, einem Behälter, mit dem der Filter verbunden ist, oder mit dem der Filter verbunden werden kann, und Teilchen, an die die zweite Komponente fixiert worden ist, als Kit (Reagenzsatz) zur Bestimmung von Nucleinsäure in einem in einem der Ansprüche 1 bis 14 definierten Verfahren.

17. Verwendung von zwei Primern, die komplementär zu den gegenüberliegenden Polaritäten der Ziel-Nucleinsäure sind, und mit einem Marker versehen sind oder versehen werden können; einer Nucleinsäure-Sonde, d.h. einer Einfangsonde, die dazu fähig ist, selektiv mit der Ziel-Nucleinsäure zu hybridisieren, und die mit einer Stelle oder einer ersten Komponente versehen ist, oder mit einer ersten Komponente versehen werden kann, die eine Affinität für eine zweite Komponente besitzt, und die zu irgendeinem modifizierten Primer nicht homolog ist; und von mindestens einem Filter, einem Behälter, mit dem der Filter verbunden ist oder mit dem der Filter verbunden werden kann, und Teilchen, an die die zweite Komponente fixiert worden ist, als Kit (Reagenzsatz) zur Bestimmung von Nucleinsäure in einem in einem der Ansprüche 1 bis 14 definierten Verfahren.

18. Verwendung von mindestens zwei Primern, die komplementär zu den gegenüberliegenden Polaritäten der Ziel-Nucleinsäure sind, und die mit einer Stelle oder einer ersten Komponente versehen sind oder versehen werden können, die Affinität für eine zweite Komponente zeigt; einer Nucleinsäuresonde, d.h. einer Detektorsonde, die dazu fähig ist, selektiv mit der Ziel-Nucleinsäure zu hybridisieren, und die mit einem bestimmbaren Marker versehen ist oder damit versehen werden kann, und die mit irgendeinem Primer nicht homolog ist; und mindestens einem Filter, einem Behälter, mit dem der Filter verbunden ist oder mit dem der Filter verbunden werden kann; und Teilchen, an die die zweite Komponente fixiert worden ist, als Kit (Reagenzsatz) zur Bestimmung von Nucleinsäure in einem in einem der Ansprüche 1 bis 14 definierten Verfahren.

19. Verwendung von mindestens zwei gegenseitig nicht homologer Sonden, die dazu fähig sind, mit der Ziel-Nucleinsäure zu hybridisieren, wobei mindestens eine von ihnen, die Detektorsonde, mit einem bestimmbaren Marker versehen ist oder damit versehen werden kann, und die andere, die Einfangsonde, an Teilchen fixiert ist, und mindestens einem Filter und einem Behälter, mit dem der Filter verbunden ist oder mit dem der Filter verbunden werden kann, als Kit (Reagenzsatz) zur Bestimmung von Nucleinsäure in einem in einem der Ansprüche 1 bis 14 definierten Verfahren.

20. Verwendung von zwei Primern, die komplementär zu den gegenüberliegenden Polaritäten der Ziel-Nucleinsäuren sind, und die mit einem bestimmbaren Marker versehen sind oder damit versehen werden können; einer Nucleinsäuresonde, d.h. einer Einfangsonde, die dazu fähig ist, selektiv mit der Ziel-Nucleinsäure zu hybridisieren, und die an Teilchen fixiert ist, und mit irgendeinem Primer nicht homolog ist; und von mindestens einem Filter und einem Behälter, mit dem der Filter verbunden ist oder mit dem der Filter verbunden werden kann; und von Teilchen, an die die Einfangsonde fixiert worden ist, als Kit (Reagenzsatz) zur Bestimmung von Nucleinsäure in einem in einem der Ansprüche 1 bis 14 definierten Verfahren.

21. Zur Verwendung zur Bestimmung von Nucleinsäure in einem Verfahren nach einem der Ansprüche 1 bis 14 geeigneter Kit (Reagenzsatz), umfassend eine Injektionsspritze, die Teilchen enthält, die dazu fähig sind, einen Hybrid/Teilchen-Komplex mit einem Hybrid der Ziel-Nucleinsäure auszubilden, und mindestens ein Filter, das mit dem Behälter verbunden ist oder mit ihm verbunden werden kann.

## Revendications

1. Procédé de dosage d'acide nucléique par hybridation, comprenant les étapes consistant
(a) à filtrer dans un récipient une solution dans laquelle on doit mettre en évidence la présence d'un acide nucléique cible ou d'une copie de la molécule d'acide nucléique cible, afin de débarrasser la solution de ses impuretés insolubles;
(b) à préparer, dans ledit récipient, à partir du filtrat, un conjugué d'une particule insoluble et de l'acide nucléique cible hybridé ou d'une copie hybridée de la molécule d'acide nucléique cible, l'acide nucléique ou la copie hybridés étant liés à la particule par l'intermédiaire d'une sonde d'acide nucléique ou, éventuellement, dans le cas d'une copie hybridée, par l'intermédiaire d'une amorce ayant servi à la synthèse de la copie;
(c) à séparer, dans ledit récipient le conjugué de la sonde et/ou de l'amorce en excès par filtration et lavage, et
(d) à déterminer la quantité de conjugué présent.

2. Procédé conforme à la revendication 1 dans lequel le récipient est une seringue.

3. Procédé conforme à la revendication 2 dans lequel la filtration se fait à travers un filtre fixé sur l'orifice de la seringue.

4. Procédé conforme à la revendication 1, 2 ou 3 comprenant les étapes consistant
(a) à hybrider l'acide nucléique cible, s'il est présent dans la solution, avec une sonde de capture et une sonde de détection et à filtrer la solution d'hybridation, à récupérer éventuellement les hybrides restant dans le filtre par rinçage du filtre,
(b) à fixer l'hybride contenu dans le filtrat sur une particule insoluble pour former un conjugué hybride-particule,
(c) à séparer le conjugué hybride-particule de la solution par filtration et lavage du conjugué afin d'éliminer toute trace de sonde de détection non hybridée,
(d) à déterminer la quantité de conjugué présent.

5. Procédé conforme à la revendication 1, 2 ou 3, comprenant les étapes consistant
(a) à filtrer la solution dans laquelle on doit mettre en évidence la présence d'un acide nucléique cible, à récupérer éventuellement l'acide nucléique cible restant dans le filtre par rinçage du filtre, puis à hybrider l'acide nucléique cible, s'il est présent dans le filtrat, avec une sonde de capture et une sonde de détection,
(b) à fixer l'hybride contenu dans le filtrat sur une particule insoluble pour former un conjugué hybride-particule,
(c) à séparer le conjugué hybride-particule de la solution par filtration et lavage du conjugué afin d'éliminer la sonde de détection non hybridée, et
(d) à déterminer la quantité de conjugué présent.

6. Procédé conforme à la revendication 1, 2 ou 3 comprenant les étapes consistant
(a1) à faire des copies de tout acide nucléique cible présent en utilisant deux amorces qui sont complémentaires aux brins de polarité opposée de l'acide nucléique cible et qui sont marquées ou sont susceptibles d'être marquées avec un marqueur détectable;
(a2) à hybrider les copies de l'acide nucléique cible avec une sonde de capture qui n'a pas de structure homologue à celles des amorces,
(a3) à filtrer la solution d'hybridation afin d'éliminer des impuretés insolubles, à récupérer éventuellement l'hybride restant dans le filtre par rinçage du filtre,
(b) à fixer l'hybride contenu dans le filtrat sur une particule insoluble par l'intermédiaire de la sonde de capture pour former un conjugué hybride-particule,
(c) à séparer le conjugué hybride-particule de la solution par filtration et lavage du conjugué pour éliminer toute trace d'amorce non hybridée; et
(d) à déterminer la quantité de conjugué présent.

7. Procédé conforme à la revendication 1, 2 ou 3 comprenant les étapes consistant
(a1) à faire des copies de tout acide nucléique cible présent cn utilisant deux amorces qui sont complémentaires aux brins de polarité opposée de l'acide nucléique cible et qui sont marquées ou sont susceptibles d'être marquées par un marqueur détectable;
(a2) à filtrer la solution contenant les copies d'acide nucléique cible pour éliminer les impuretés insolubles, à récupérer éventuellement les copies d'acide nucléique cible restant dans le filtre par rinçage du filtre;
(a3) à hybrider les copies de l'acide nucléique cible contenues dans le filtrat avec une sonde de capture qui n'a pas de structure homologue à celles des amorces,
(b) à fixer l'hybride sur une particule insoluble par l'intermédiaire de la sonde de capture pour former un conjugué hybride-particule,
(c) à séparer le conjugué hybride-particule de la solution par filtration et lavage du conjugué pour éliminer toute trace d'amorce non hybridée; et
(d) à déterminer la quantité de conjugué présent.

8. Procédé conforme à la revendication 1, 2 ou 3 comprenant les étapes consistant
(a1) à faire des copies de tout acide nucléique cible présent en utilisant deux amorces qui sont complémentaires aux brins de polarité opposée de l'acide nucléique cible;
(a2) à hybrider les copies de l'acide nucléique cible avec une sonde de détection marquée ou susceptible d'être marquée avec un marqueur détectable et qui n'a pas de structure homologue à celles des amorces,
(a3) à filtrer la solution d'hybridation afin d'éliminer les impuretés insolubles, à récupérer éventuellement l'hybride restant dans le filtre par rinçage du filtre,
(b) à fixer l'hybride sur une particule insoluble par l'intermédiaire d'une amorce pour former un conjugué hybride-particule,
(c) à séparer le conjugué hybride-particule de la solution par filtration et lavage du conjugué pour éliminer toute trace de sonde de détection non hybridée; et
(d) à déterminer la quantité de conjugué présent.

9. Procédé conforme à la revendication 1, 2 ou 3 comprenant les étapes consistant
(a1) à faire des copies de tout acide nucléique cible présent en utilisant deux amorces qui sont complémentaires aux brins de polarité opposée de l'acide nucléique cible;
(a2) à filtrer la solution contenant les copies de l'acide nucléique cible pour éliminer les impuretés insolubles, à récupérer éventuellement les copies de l'acide nucléique cible restant dans le filtre par rinçage du filtre;
(a3) à hybrider les copies de l'acide nucléique cible contenues dans le filtrat avec une sonde de détection marquée ou susceptible d'être marquée avec un marqueur détectable et qui n'a pas de structure homologue à celles des amorces,
(b) à fixer l'hybride sur une particule insoluble par l'intermédiaire d'une amorce pour former un conjugué hybride-particule,
(c) à séparer le conjugué hybride-particule de la solution par filtration et lavage du conjugué pour éliminer toute trace de sonde de détection non hybridée; et
(d) à déterminer la quantité de conjugué présent.

10. Procédé conforme à une quelconque des revendications 1 à 3 comprenant les étapes consistant
(a) à filtrer une solution contenant une sonde de détection et dans laquelle il s'agit de mettre en évidence la présence d'un acide nucléique cible, à récupérer éventuellement l'acide nucléique cible restant dans le filtre par rinçage du filtre,
(b) à hybrider l'acide nucléique cible, s'il est présent dans le filtrat, avec une sonde de détection et une sonde de capture, la sonde de capture étant fixée sur une particule insoluble, dans le but de former un conjugué hybride-particule,
(c) à séparer le conjugué hybride-particule de la solution par filtration et lavage du conjugué afin d'éliminer la sonde de détection non hybridée, et
(d) à déterminer la quantité de conjugué présent.

11. Procédé conforme à une quelconque des revendications 1 à 3, comprenant les étapes consistant
(a) à hybrider au préalable l'acide nucléique cible, s'il est présent dans la solution, avec une sonde de détection et à filtrer la solution d'hybridation, à récupérer éventuellement l'hybride restant dans le filtre par rinçage du filtre,
(b) à hybrider l'acide nucléique préalablement hybridé avec une sonde de capture, la sonde de capture étant fixée à une particule insoluble, dans le but de former un conjugué hybride-particule,
(c) à séparer le conjugué hybride-particule de la solution par filtration et lavage du conjugué afin d'éliminer la sonde de détection non hybridée, et
(d) à déterminer la quantité de conjugué présent.

12. Procédé conforme à une quelconque des revendications 1 à 3 comprenant les étapes consistant
(a1) à faire des copies de tout acide nucléique cible présent en utilisant deux amorces qui sont complémentaires aux brins de polarité opposée de l'acide nucléique cible et qui sont marquées ou sont susceptibles d'être marquées par un marqueur détectable;
(a2) à filtrer la solution contenant les copies de l'acide nucléique cible pour éliminer les impuretés insolubles, à récupérer éventuellement les copies de l'acide nucléique cible restant dans le filtrc par rinçage du filtre;
(b) à hybrider les copies de l'acide nucléique cible, si elles sont présentes dans le filtrat, avec une sonde d'acide nucléique qui n'a pas de structure homologue à celle des amorces et qui est fixée à une particule insoluble, pour former un conjugué hybride-particule;
(c) à séparer le conjugué hybride-particule de la solution par filtration et lavage du conjugué pour éliminer toute trace d'amorce non hybridée; et
(d) à déterminer la quantité de conjugué présent.

13. Procédé conforme à une quelconque des revendications 4, 5, 8, 9, 10 ou 11 dans lequel, dans l'étape (d), la sonde de détection est éluée du conjugué hybride-particule et est ensuite dosée.

14. Procédé conforme à une quelconque des revendications 6, 7 ou 12 dans lequel, dans l'étape (d), on élue les copies de l'acide nucléique cible pourvues d'une amorce marquée du conjugué hybride particule puis on dose la quantité de marqueur dans le surnageant.

15. Utilisation d'un récipient contenant des particules capables de former avec un hybride de l'acide nucléique cible un complexe hybride-particule et d'au moins un filtre qui est connecté ou peut être connecté au récipient, sous forme d'un kit pour un procédé de dosage défini dans une quelconque des revendications 1 à 14.

16. Utilisation d'au moins deux sondes non homologues l'une à l'autre, capables de s'hybrider avec l'acide nucléique cible, au moins une des sondes, la sonde de détection, étant marquée ou pouvant être marquée par un marqueur détectable, et au moins une des sondes, la sonde de capture, étant pourvue d'un site ou d'un premier composant, ou pouvant être pourvue d'un premier composant, ayant une affinité pour un deuxième composant, d'au moins un filtre, d'un récipient auquel le filtre est fixé ou auquel il peut être fixé, et de particules auxquelles ledit deuxième composant a été fixé, sous forme d'un kit de réactifs pour le dosage d'acide nucléique dans un procédé conforme à une quelconque des revendications 1 à 14.

17. Utilisation de deux amorces, qui sont complémentaires aux brins de polarité opposée de l'acide nucléique cible et qui sont marquées ou susceptibles d'être marquées avec un marqueur détectable, d'une sonde d'acide nucléique, c'est-à-dire d'une sonde de capture, qui est capable de s'hybrider sélectivement avec l'acide nucléique cible et qui est pourvue d'un site ou d'un premier composant, ou qui est capable d'être pourvue d'un premier composant ayant une affinité pour un second composant, et qui n'est pas homologue aux amorces modifiées, d'au moins un filtre, d'un récipient auquel le filtre est fixé ou auquel il peut être fixé, et de particules auxquelles ledit deuxième composant a été fixé, sous forme d'un kit de réactifs pour le dosage d'acide nucléique dans un procédé conforme à une quelconque des revendications 1 à 14.

18. Utilisation d'au moins deux amorces qui sont complémentaires aux brins de polarité opposée de l'acide nucléique cible et qui sont pourvues ou peuvent être pourvues d'un site ou d'un premier composant ayant une affinité pour un second composant, d'une sonde d'acide nucléique, c'est-à-dire d'une sonde de détection qui est capable de s'hybrider sélectivement avec l'acide nucléique cible et qui est marquée ou peut être marquée par un marqueur détectable, et qui n'est pas homologue aux amorces, d'au moins un filtre, d'un récipient auquel le filtre est fixé ou auquel il peut être fixé, et de particules auxquelles ledit deuxième composant a été fixé, sous forme d'un kit de réactifs pour le dosage d'acide nucléique dans un procédé conforme à une quelconque des revendications 1 à 14.

19. Utilisation d'au moins deux amorces, non homologues l'une à l'autre, capables de s'hybrider avec l'acide nucléique cible, au moins une d'entre elles, à savoir la sonde de détection, étant marquée ou pouvant être marquée avec un marqueur détectable, et l'autre, à savoir la sonde de capture, étant fixée à des particules, d'au moins un filtre et d'un récipient auquel le filtre est fixé ou peut être fixé sous forme d'un kit de réactifs pour le dosage d'acide nucléique dans un procédé conforme à une quelconque des revendications 1 à 14.

20. Utilisation de deux amorces complémentaires aux brins de polarité opposé de l'acide nucléique cible et qui sont marquées ou susceptibles d'être marquées par un marqueur détectable d'une sonde d'acide nucléique, c'est-à-dire d'une sonde de capture, qui est capable de s'hybrider sélectivement avec l'acide nucléique cible et qui est fixée à des particules et n'est pas homologue aux amorces, d'au moins un filtre, d'un récipient auquel le filtre est fixé ou auquel il peut être fixé, et de particules auxquelles ladite sonde de capture a été fixée, sous forme d'un kit de réactifs pour le dosage d'acide nucléique dans un procédé conforme à une quelconque des revendications 1 à 14.

21. Kit pouvant être utilisé pour le dosage d'acide nucléique dans un procédé conforme à une quelconque des revendications 1 à 14, comprenant une seringue contenant des particules capables de former avec un hybride de l'acide nucléique cible un complexe hybride particule, et au moins un filtre qui est fixé ou peut être fixé sur la seringue.
